# EUROPEAN PATENT APPLICATION

(11) **EP 3 199 086 A1**
(43) Date of publication of application: **02.08.2017**
(21) Application number: 16802888.4
(22) Date of filing: 25.03.2016
(51) Int. Cl.: A61B 1/00, A61B 1/04, G02B 23/24

(54) **ENDOSCOPE**

(30) Priority: 02.06.2015 JP 2015112482
(71) Applicant: Olympus Corporation, Tokyo 192-8507 (JP)
(72) Inventor: MATSUO, Naomi, Tokyo 192-8507 (JP)
(74) Representative: Schicker, Silvia
(86) International application number: PCT/JP2016/059592
(87) International publication number: WO 2016/194450

(57) **Abstract**

An endoscope according to the present invention includes an elongated insertion section that is inserted into a subject, an attachment portion that is provided on the insertion section, and to which a circular cylindrical external appliance is attached, and a locking member that is disposed on a more distal end side of the insertion section than the attachment portion, and that prevents removal of the external appliance from the insertion section by abutting against a surface, of the external appliance, facing a distal end.

## Description

### Technical Field

The present invention relates to an endoscope which prevents removal of a detachable external appliance attached to an insertion section.

### Background Art

Conventionally, endoscopes are known which enable observation of an inside of a subject by insertion of an elongated insertion section into the subject, and which enable various treatments and procedures as necessary by insertion of a treatment instrument into the subject through a treatment instrument insertion channel provided in the insertion section.

Generally, an endoscope including an elongated insertion section is provided with a bending portion at a distal end side of the insertion section, and the bending portion may be bent in an up-down direction and a left-right direction by operation of a bending operation knob provided on a hand side. At a time of inserting the insertion section of the endoscope into a complicated subject, such as a lumen which makes a 360-degree loop as exemplified by a large intestine of a human, a surgeon guides an insertion section distal end to an observation target region by bending the bending portion by operating the bending operation knob and turning the insertion section.

However, a high level of skill is necessary to smoothly guide the insertion section deep into a complicated lumen such as a large intestine. Accordingly, various techniques are proposed so as to allow the insertion section to be smoothly moved forward and backward in a lumen by attaching an external appliance to the insertion section and by operation of the external appliance.

For example, Japanese Patent No. 5326049 discloses a technique for improving insertability of an endoscope insertion section by attaching an external appliance (attachment unit), which is capable of rotating around an axis, to the endoscope insertion section, rotating the external appliance to cause a spiral fin portion provided on an outer circumference of the external appliance to contact an inner wall of a subject, and thereby generating a propulsive force in the axial direction with respect to the insertion section.

After the insertion section of the endoscope has been guided to an observation target region by being moved forward/backward in a predetermined manner in the subject and has reached the observation target region, an operation of extracting the insertion section to outside is performed. According to the structure disclosed in Japanese Patent No. 5326049, when moving the insertion section forward by rotating the external appliance in an insertion direction, a force is applied to the external appliance to cause the external appliance to move to the distal end side of the insertion section. On the other hand, when moving the insertion section backward by rotating the external appliance in a backward direction, a force is applied to the external appliance to cause the external appliance to move to a distal end side of the insertion section.

Even if engagement of the external appliance and the insertion section is released and the external appliance is moved to the proximal end side of the insertion section, the external appliance is not removed from the insertion section. However, when the external appliance is moved in the insertion section distal end direction, the external appliance is possibly removed from the distal end side of the insertion section.

To cope with such an event, it is conceivable to tightly fix the external appliance to the insertion section, but if the external appliance is tightly fixed, detachment is made difficult, and the operability is reduced.

In view of the above circumstance, the present invention has an object to provide an endoscope according to which an external appliance does not have to be tightly fixed to an insertion section, and according to which the external appliance is not removed even if engagement of the insertion section and the external appliance is released and the external appliance is moved to the endoscope distal end side.

### Disclosure of Invention

### Means for Solving the Problem

An endoscope according to an aspect of the present invention includes an elongated insertion section that is inserted into a subject, an attachment portion that is provided on the insertion section, and to which a circular cylindrical external appliance is attached, and a locking member that is disposed on a more distal end side of the insertion section than the attachment portion, and configured to prevent removal of the external appliance from the insertion section by abutting against a surface, of the external appliance, facing a distal end.

### Brief Description of the Drawings

Fig. 1 is a perspective view of an endoscope according to a first embodiment;
Fig. 2A is a cross-sectional view, according to the first embodiment, of a state of attaching an external appliance to an insertion section;
Fig. 2B is a cross-sectional view, according to the first embodiment, of a state where the external appliance is attached to the insertion section;
Fig. 3 is a schematic view, according to the first embodiment, showing a relationship among outer diameters of respective parts of the endoscope and inner diameters of respective parts of the external appliance;
Fig. 4 is a schematic view, according to a second embodiment, showing a relationship among outer diameters of respective parts of an endoscope and inner diameters of respective parts of an external appliance;
Fig. 5 is a perspective view, according to a third embodiment, of a state of attaching an external appliance to an insertion section of an endoscope;
Fig. 6 is a front view, according to the third embodiment, of a state where the external appliance is attached to the insertion section;
Fig. 7 is a cross-sectional view, according to the third embodiment, taken along VII-VII in Fig. 6;
Fig. 8 is a front view, according to a fourth embodiment, of a state of attaching an external appliance to an insertion section;
Fig. 9 is a front view, according to a fifth embodiment, of a state of attaching an external appliance to an insertion section;
Fig. 10 is a perspective view, according to the fifth embodiment, of a state of attaching the external appliance to the insertion section;
Fig. 11 is a schematic view, according to a sixth embodiment, showing a positional relationship among a locking member and an attachment portion provided on an insertion section and an external appliance; and
Fig. 12 is an enlarged view of a portion XII in Fig. 11.

### Best Mode for Carrying Out the Invention

Hereinafter, an embodiment of the present invention will be described with reference to the drawings.

### [First Embodiment]

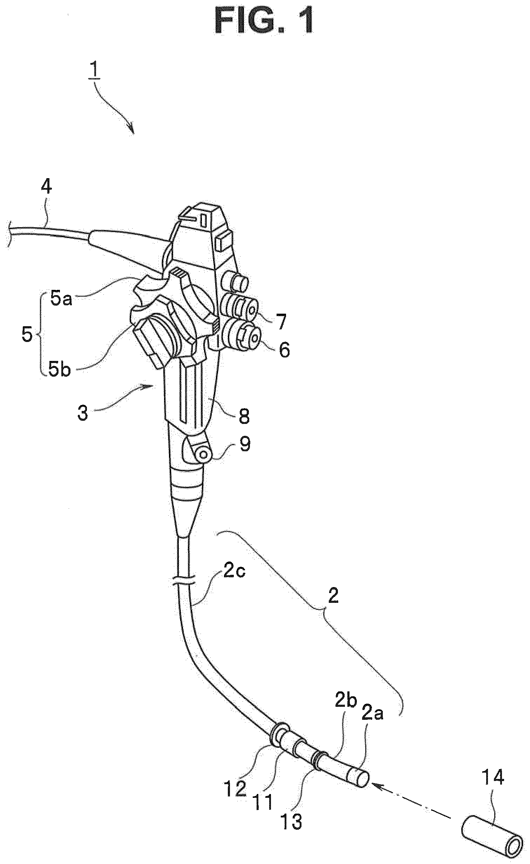

A first embodiment of the present invention is shown in Figs. 1 to 3. As shown in Fig. 1, an endoscope 1 includes an elongated insertion section 2, an operation section 3 which is provided continuously on a proximal end side of the insertion section 2, and a universal cable 4 which extends from the operation section 3, and a distal end portion 2a as a distal end rigid portion, a bending portion 2b and a flexible tube portion 2c are continuously provided in the insertion section 2, in the above order, from the distal end side.

The operation section 3 is provided with a bending operation knob 5 configured to perform bending operation of the bending portion 2b of the insertion section 2, and also with switches and the like 6, 7 used to perform various functions of the endoscope. Furthermore, the bending operation knob 5 includes an UD bending operation knob 5a used for bending the bending portion 2b in an up-down direction, and an RL bending operation knob 5b used for bending the bending portion 2b in a left-right direction. Moreover, a grasping section 8 is provided at a coupling portion of the insertion section 2 and the operation section 3, and an opening section 9 of a treatment instrument channel is provided in a bend preventing portion which is provided between the grasping section 8 and one end of the flexible tube portion 2c. Note that, although not shown, an extending end of the universal cable 4 is connected to a peripheral device such as a light source device or a video processor.

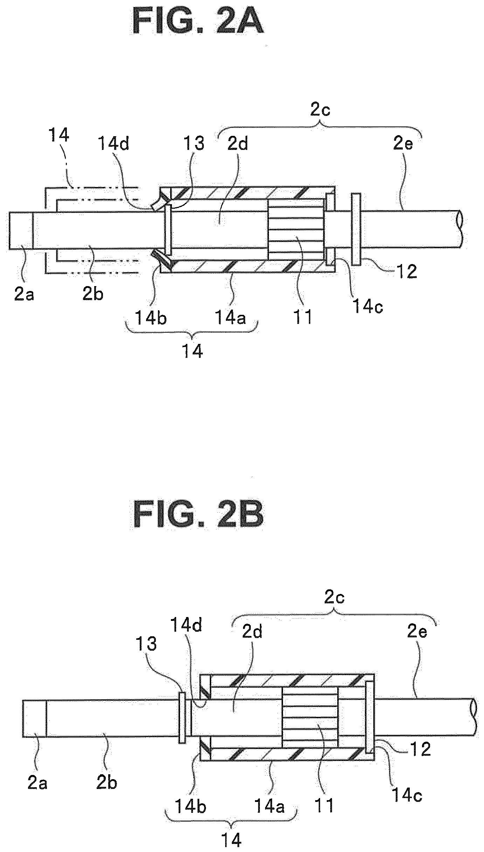

Furthermore, as shown in Fig. 2, the flexible tube portion 2c is divided into a first flexible tube portion 2d which is provided continuously on the proximal end side of the bending portion 2b, and a second flexible tube portion 2e which is provided continuously on a proximal end side of the first flexible tube portion 2d, and both of the flexible tube portions 2d, 2e are joined to each other by a metal pipe sleeve (not shown). Moreover, an attachment portion 11 is provided on the outer circumference of the pipe sleeve. The attachment portion 11 is a rotating body that rotates around the axis core of the pipe sleeve, for example, and is made rotatable by being provided continuously in a rotation drive section (not shown) provided in the pipe sleeve.

Moreover, a flange-shaped fixing portion 12 is fixedly provided on the second flexible tube portion 2e disposed on the proximal end side of the attachment portion 11, at a position facing the attachment portion 11 with a predetermined gap to the attachment portion 11. On the other hand, a flange-shaped locking member 13 is fixedly provided on a proximal portion of the bending portion 2b. Note that the fixing portion 12 and the locking member 13 are made of resin, for example, and have certain levels of rigidity.

Moreover, an external appliance 14 is attached to the attachment portion 11. The external appliance 14 is detachable, and includes an external appliance main body 14a which has a circular cylindrical shape, and an annular locked member 14b which is fixedly provided on a front end of the external appliance main body 14a. The external appliance main body 14a is made of resin and is relatively rigid, and the locked member 14b is formed of an elastic member such as a resin foam material or rubber. Furthermore, the inner circumference of the external appliance main body 14a and the outer circumference of the attachment portion 11 are engaged while being restricted with respect to a rotation direction by a spline, a key or the like.

Furthermore, a circular step portion 14c which is fitted with the fixing portion 12 is formed at a rear end portion of the external appliance main body 14a. The inner circumference of the step portion 14c is detachably and slidably engaged with the outer circumference of the fixing portion 12 while being restricted with respect to movement in the axial direction. As the engagement structure, a structure is conceivable which allows sliding in the rotation direction while restricting movement in the axial direction by forming a circular groove to one of the inner circumference of the step portion 14c and the outer circumference of the fixing portion 12, forming a circular claw portion to the other of the inner circumference of the step portion 14c and the outer circumference of the fixing portion 12, and engaging the claw portion with the circular groove. Note that, although not shown, a fin which generates a thrust force by rotation of the external appliance 14 is spirally formed on the outer circumference of the external appliance main body 14a, for example.

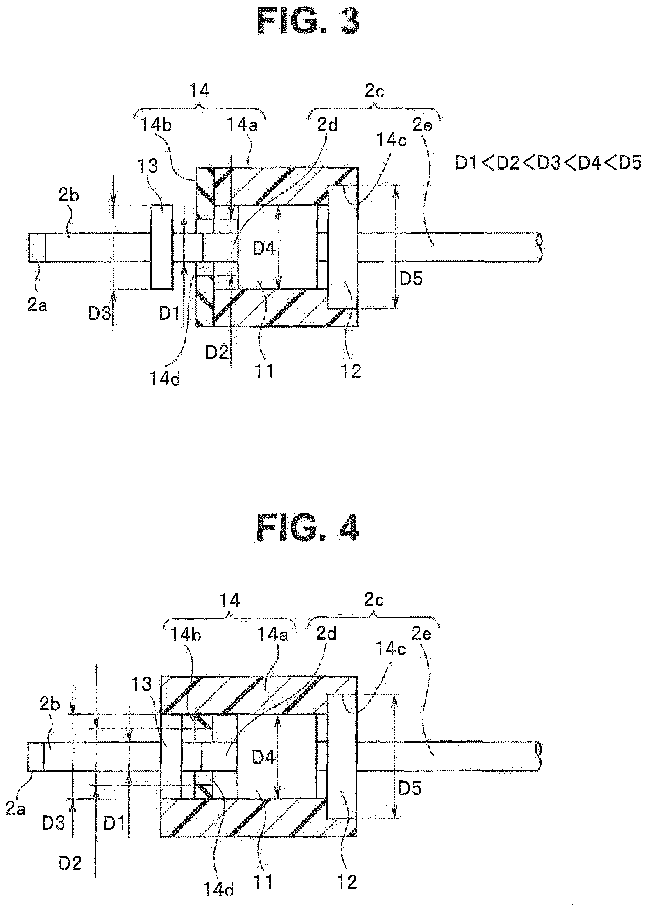

Moreover, Fig. 3 shows a relationship among an outer diameter D1 of the distal end side of the insertion section 2, an outer diameter D3 of the locking member 13, and an outer diameter D5 of the fixing portion 12, and a minimum inner diameter D2 of a through hole 14d formed in the axial core of the locked member 14b of the external appliance 14, and an inner diameter D4 of the external appliance main body 14a. Note that the inner diameter of the step portion 14c is approximately the same as the outer diameter D5 of the fixing portion 12. Accordingly, the inner diameter of the step portion 14c is a maximum inner diameter portion of the external appliance 14.

Next, an action of the present embodiment according to such a configuration will be described. As shown in Fig. 1, the external appliance 14 is attached to the insertion section 2 of the endoscope 1 from the front side. That is, the rear end of the external appliance main body 14a constituting the external appliance 14 is inserted over the distal end portion 2a of the insertion section 2 and is moved in the direction of the first flexible tube portion 2d. As shown in Fig. 3, the inner diameter D4 of the external appliance main body 14a and the inner diameter D5 of the circular step portion 14c formed at the rear end of the external appliance main body 14a are larger than the outer diameter D4 of the locking member 13 formed at a rear portion of the bending portion 2b (D3 > D4 > D5), and thus, as shown by a dashed-two dotted line in Fig. 2A, the external appliance main body 14a may be inserted to the first flexible tube portion 2d side without interfering with the locking member 13.

Then, when the inner surface of the locked member 14b, which is fixedly provided at the distal end of the external appliance main body 14a, abuts against the locking member 13, movement in the direction of the first flexible tube portion 2d is interrupted due to the minimum inner diameter D2 of the through hole 14d piercing the locked member 14b being smaller than the outer diameter D3 of the locking member 13 (D2 < D3), as shown in Fig. 3. However, the locked member 14b is formed of an elastic member, and when a certain pulling force is applied, the through hole 14d is deformed in such a way that the diameter is increased along the outer circumference of the locking member 13 so as to allow the locking member 13 to pass through, as shown in Fig. 2A.

Note that the pulling force for deforming and enlarging the diameter of the through hole 14d piercing the locked portion 14b is set to a greater value than a force in the axial direction that is generated at the external appliance 14 at the time of pulling or pushing of the insertion section 2 of the endoscope 1 inserted in a subject.

Then, as shown in Fig. 2B, when the through hole 14d lets pass the locking member 13, the locked member 14b is restored to the initial shape, and the front surface of the locked member 14b faces the locking member 13, the circular step portion 14c formed at the rear end is engaged with the outer circumference of the fixing portion 12 formed on the second flexible tube portion 2e, and movement in the axial direction is restricted. Moreover, the inner circumference of the external appliance main body 14a is engaged with the outer circumference of the attachment portion 11 formed on the first flexible tube portion 2d by a spline, a key or the like, and movement in the rotation direction is thereby restricted.

On the other hand, in the case of taking off the external appliance 14 from the insertion section 2 of the endoscope 1, the external appliance 14 can be easily taken off by pulling the external appliance main body 14a in the direction of the distal end portion 2a of the insertion section 2, separating the step portion 14c from the fixing portion 12, and elastically deforming the locked member 14b provided at the distal end so as to allow the locking member 13 to pass through.

The external appliance 14 attached to the insertion section 2 operates in an integrated manner with the attachment portion 11, and in the case where the attachment portion 11 is a rotating body that is rotated by a rotation drive section, not shown, for example, the external appliance 14 is rotated in an integrated manner under the rotation force from the attachment portion 11, and a propulsive force is generated by the spiral fin provided on the outer circumference.

When rotation by a propulsive force acting in the direction of causing the external appliance 14 to move the endoscope 1 forward is taken as forward rotation, and rotation by a propulsive force acting in the direction of causing the external appliance 14 to move the endoscope 1 backward is taken as reverse rotation, if the external appliance 14 is reversely rotated and the insertion section 2 is moved backward, a reaction force in the backward direction is applied to external appliance 14. However, because the circular step portion 14c formed at the rear end of the external appliance main body 14a is locked with the fixing portion 12, the external appliance 14 does not move over the fixing portion 12 to the second flexible tube portion 2e side. Even if the step portion 14c of the external appliance 14 moves over the fixing portion 12 to the proximal end side, the external appliance 14 moves to the side of the operation section 3, which is continuous to the proximal end side of the insertion section 2, and is not removed from the insertion section 2.

On the other hand, in the case of forwardly rotating the external appliance 14 and causing the insertion section 2 to move forward, a reaction force in the forward direction is applied to the external appliance 14. When the reaction force exceeds the engaging force between the step portion 14c formed at the rear end of the external appliance main body 14a and the fixing portion 12, the engagement is released, and the external appliance 14 is moved to the distal end side of the insertion section 2.

When the external appliance 14 moves in the distal end direction, the locked member 14b abuts against the locking member 13. However, the minimum inner diameter D2 of the through hole 14d piercing the locked member 14b is smaller than the outer diameter D3 of the locking member 13, and thus, the locked member 14b does not move forward than the locking member 13, and the external appliance 14 is not removed from the distal end portion 2a of the insertion section 2.

Accordingly, when the distal end portion 2a of the endoscope 1 is inserted into a subject, such as a body cavity of a human body, even if engagement of the step portion 14c of the external appliance 14 and the fixing portion 12 is released, the external appliance 14 is not removed from the insertion section 2, and is not left inside the subject.

As described above, according to the present embodiment, the locked member 14b of the external appliance 14 is an elastic member, and the locked member 14b is locked with the locking member 13 formed on the distal end side of the insertion section 2 so as to be retained, and thus, the external appliance 14 can be easily detached from the distal end portion 2a side of the endoscope 1, and the handling property is increased.

Furthermore, the external appliance does not have to be tightly fixed to the insertion section, and in the case of repeatedly using the external appliance by cleaning after use or in the case where the external appliance is disposable, the structure can be simplified due to the structure allowing easy detachment.

Note that the locking member 14b described above may be formed of an elastic member, or the locked member 14b may be integrally formed with the external appliance main body 14a and only the locking member 14b may be formed of an elastic member.

### [Second Embodiment]

A second embodiment of the present invention is shown in Fig. 4. Note that the same structural components as the structural components of the first embodiment are denoted by the same reference signs, and description of the structural components is omitted.

In the first embodiment described above, the locked member 14b is provided at a front end of the external appliance main body 14a of the external appliance 14, but in the present embodiment, the locked member 14b is located inside the external appliance main body 14a, that is, at a position slightly rearward from the front end of the external appliance main body 14a.

As shown in Fig. 4, by providing the locked member 14b at a position slightly rearward from the distal end of the external appliance main body 14a, the locking member 13 may be housed inside the external appliance main body 14a. As a result, the locking member 13 may be prevented from interfering with an inner wall of a subject, and both the inner wall of the subject and the locking member 13 can be protected.

### [Third Embodiment]

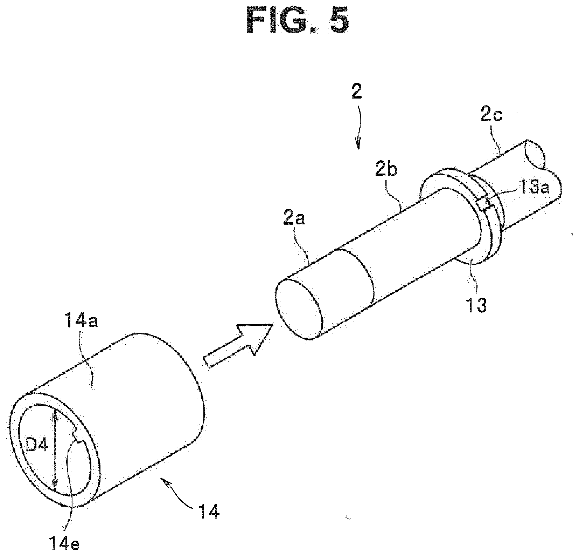

A third embodiment of the present invention is shown in Figs. 5 to 7. Note that the same structural components as the structural components of the first embodiment are denoted by the same reference signs, and detailed description of the structural components is omitted.

In the first embodiment described above, the locked member 14b provided at a front surface of the external appliance main body 14a is made of an elastic member, and at the time of attachment, the locked member 14b is elastically deformed so as to move over the locking member 13, but in the present embodiment, a recessed portion 13a is formed in the locking member 13, and a claw-shaped locked portion 14e capable of passing the recessed portion 13a is formed to a front surface of the external appliance 14.

That is, as shown in Fig. 5, the claw-shaped locked portion 14e is integrally formed at the front end of the external appliance 14, and thus, the locked portion 14e and the external appliance main body 14a are made of the same material.

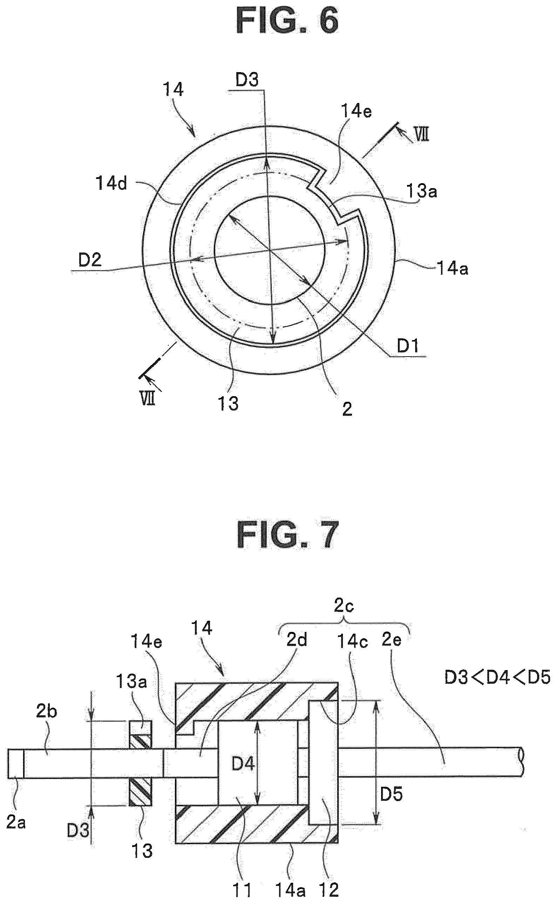

Also, as shown in Figs. 6 and 7, an inner shape of the front surface of the external appliance 14 and an outer shape of the locking member 13 which is inserted through the front surface are formed into substantially similar shapes, and also, a minimum inner diameter D2 of the locked portion 14e is formed to be smaller than the outer diameter D3 of the locking member 13.

According to such a structure, at the time of attaching the external appliance 14 to the insertion section 2 of the endoscope 1 from the distal end portion 2a side, first, the claw-shaped locked portion 14e formed on the external appliance 14 is positioned with respect to the recessed portion 13a formed in the locking member 13. Then, in the present state, the step portion 14c side of the external appliance main body 14a is inserted over the distal end portion 2a.

Then, the claw-shaped locked portion 14e passes the recessed portion 13a formed in the locking member 13, and the inner circumference of the external appliance main body 14a is engaged with the outer circumference of the attachment portion 11 by a spline or a key, and the step portion 14c formed at the rear end is engaged with the fixing portion 12 in a state where sliding in the rotation direction is allowed and movement in the axial direction is restricted.

The attachment portion 11 is a rotating body, and when the external appliance 14 is integrally rotated with the attachment portion 11, phases of the locked member 14e and the recessed portion 13a are shifted from each other. Accordingly, even if the external appliance 14 comes off the fixing portion 12 and moves in the direction of the distal end portion 2a of the insertion section 2, the locked portion 14e is locked with the locking member 13, and removal from the distal end portion 2a is prevented.

In the present embodiment, the locked member 14e is integrally formed with the external appliance main body 14a, and manufacture can be facilitated. Also, the external appliance 14 is not deteriorated even when repeatedly attached to and detached from the insertion section 2, and high durability is achieved.

### [Fourth Embodiment]

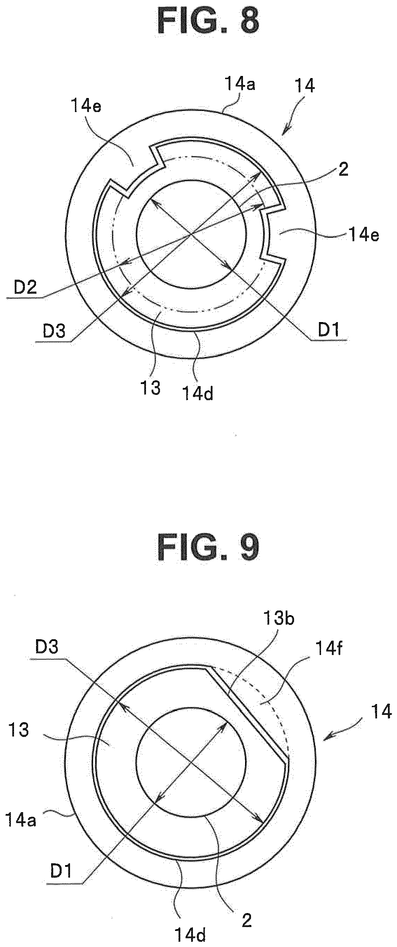

A fourth embodiment of the present invention is shown in Fig. 8. The present embodiment is a modification of the third embodiment. In the third embodiment described above, the recessed portion 13a and the locked portion 14e are formed at one position of the outer circumference of the locking portion 13 and one position of the distal end of the external appliance 14, respectively, but in the present embodiment, the recessed portions 13a and the locked portions 14e are formed at a plurality of positions (two in the drawing).

As indicated by the present embodiment, when the recessed portions 13a and the locked portions 14e with aligned phases are formed at a plurality of positions (two in the drawing), a plurality of locked portions 14e are locked with the locking member 13, and removal of the external appliance 14 may be more reliably prevented than in the third embodiment described above.

### [Fifth Embodiment]

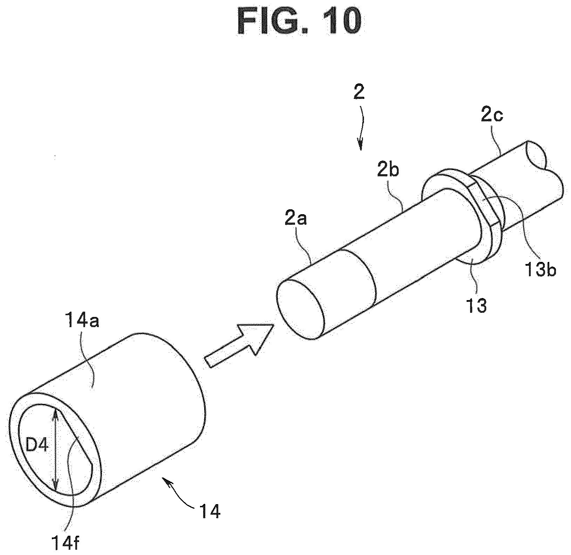

A fifth embodiment of the present invention is shown in Figs. 9 and 10. Note that the same structural components as the structural components of the first embodiment are denoted by the same reference signs, and description of the structural components is omitted.

In the present embodiment, the opening shape of a distal end surface of the external appliance 14 is D-shaped with a part of a circle formed into a chordal shape, and the chordal part is made a locked portion 14f. Moreover, a part of the locking member 13 corresponding to the locked portion 14f is cut flatly into an escape portion 13b, and as shown in Fig. 9, the outer shape of the locking member 13 and the inner shape of the distal end of the external appliance 14 are formed into similar shapes.

Accordingly, by aligning the phases of the distal end opening of the external appliance 14 and the locking member 13, the external appliance 14 can be easily attached to and detached from the insertion section 2 of the endoscope 1. Also, when the attached external appliance 14 is rotated by the attachment portion 11, the phases of the locked portion 14f and the escape portion 13b of the locking portion 13 are shifted from each other, and even if the external appliance 14 comes off the fixing portion 12 and moves in the direction of the distal end portion 2a of the insertion section 2, removal from the distal end portion 2a is prevented because the locked portion 14e is locked with the locking member 13.

### [Sixth Embodiment]

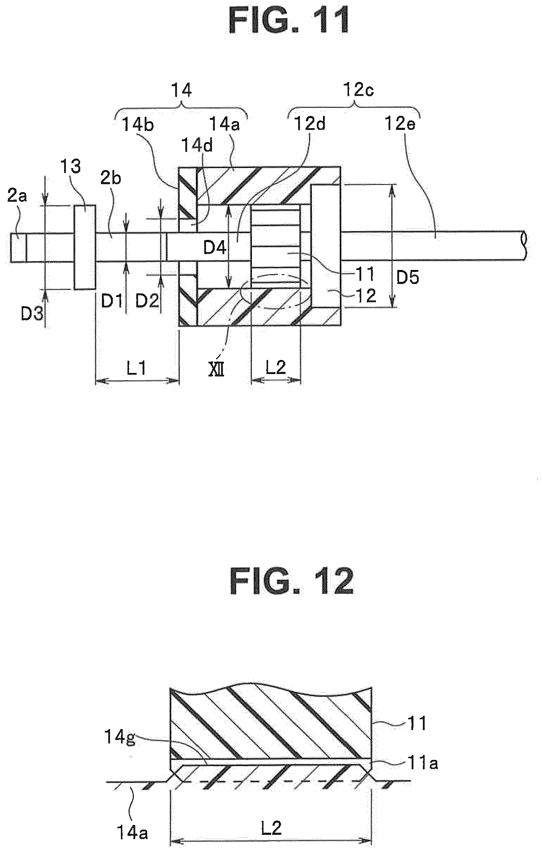

A sixth embodiment of the present invention is shown in Figs. 11 and 12. As shown in Fig. 11, in the present embodiment, a gap length L1 between the locked member 14b provided at a front portion of the external appliance 14 and the locking member 13 is made longer than an effective engagement length L2 of the attachment portion 11 (L1 > L2).

As shown in Fig. 12, in the present embodiment, tooth widths of external spline teeth 11a formed on the attachment portion 11 and internal spline teeth 14g formed on the locked member 14b are approximately the same, and accordingly, the effective engagement length L2 is approximately the same as the tooth width of the attachment portion 11.

In the present embodiment, the gap length L1 between the locked member 14b and the locking member 13 is longer than the effective engagement length L2 of the attachment portion 11, and thus, when the external appliance 14 comes off the fixing portion 12 and moves to the distal end side of the endoscope 1, and the locked member 14b is locked with the locking member 13, the internal spline teeth 14g formed on the external appliance main body 14a are removed from the external spline teeth 11 a formed on the attachment portion 11.

Accordingly, the attachment portion 11 is rotated idly, and transmission of a rotation force to the external appliance 14 is interrupted. On the other hand, in a state where coupling of the external appliance main body 14a and the attachment portion 11 is not released even in a state where the locked member 14b is locked with the locking member 13 (in a state where the dimensional relationship between L1 and L2 is L1 < L2), the through hole 14d piercing the locked member 14b may be widened causing the locking member 13 to be crossed.

In the present embodiment, the gap length L1 between the locked member 14b and the locking member 13 is made longer than the effective engagement length L2 of the attachment portion 11. Accordingly, when the locked member 14b of the external appliance 14 is locked with the locking member 13, engagement with the attachment portion 11 is released and rotation force is not transmitted, and the external appliance 14 does not move over the locking member 13, and thus, removal may be more reliably prevented.

Note that the present invention is not limited to the embodiments described above, and, for example, in the sixth embodiment, if the tooth width of the internal spline teeth 14g formed on the external appliance main body 14a is made shorter than the tooth width of the external spline teeth 11 a formed on the attachment portion 11, the effective engagement length L2 of the attachment portion 11 is shortened, and thus, the gap length L1 may be shortened. Moreover, the attachment portion 11 and the external appliance 14 may be engaged by a key.

The present application claims priority from Japanese Patent Application No. 2015-112482 filed in Japan on June 2, 2015, the entire contents of which are incorporated in the present description, claims, and drawings by reference.

## Claims

1. An endoscope comprising:
an elongated insertion section that is inserted into a subject;
an attachment portion that is provided on the insertion section, and to which a circular cylindrical external appliance is attached; and
a locking member that is disposed on a more distal end side of the insertion section than the attachment portion, and configured to prevent removal of the external appliance from the insertion section by abutting against a surface, of the external appliance, facing a distal end.

2. The endoscope according to claim 1, wherein
an outer circumference of the attachment portion is engaged with an inner circumference of the external appliance, and
a distance between a rear end surface of the locking member and the surface, of the external appliance, facing the distal end is longer than an effective engagement length of the attachment portion and the external appliance in an axial direction.

3. The endoscope according to claim 1 or 2, wherein a maximum outer diameter of the locking member is formed larger than a minimum inner diameter of the external appliance.

4. The endoscope according to claim 3, wherein the maximum outer diameter of the locking member is formed smaller than a maximum inner diameter of the external appliance.

5. The endoscope according to any one of claims 1 to 4, further comprising a fixing portion that is disposed on a more proximal end side of the insertion section than the locking member, wherein
the fixing portion is engaged with an inner circumference of the external appliance.

6. The endoscope according to any one of claims 1 to 5, wherein the locking member is made of an elastic member.

7. The endoscope according to claim 3, wherein at least one of the locking member and a part, of the external appliance, with the minimum inner diameter is made of an elastic member.

8. The endoscope according to claim 3, wherein an outer shape of the locking member and an inner shape of the external appliance are formed to have similar shapes, other than a circular shape, that allow insertion to each other.
